# EUROPEAN PATENT SPECIFICATION

(11) **EP 4 209 573 B1**
(45) Date of publication and mention of the grant of the patent: **09.12.2026**
(21) Application number: 20952366.1
(22) Date of filing: 01.09.2020
(51) Int. Cl.: C12M 1/00, C12Q 1/6806, G01N 35/00

(54) **PRETREATMENT MECHANISM-INTEGRATED NUCLEIC ACID ANALYSIS DEVICE**
VORRICHTUNG ZUR ANALYSE VON NUKLEINSÄUREN MIT INTEGRIERTEM VORBEHANDLUNGSMECHANISMUS
DISPOSITIF D'ANALYSE D'ACIDE NUCLÉIQUE À MÉCANISME DE PRE-TRAITEMENT INTÉGRÉ

(43) Date of publication of application: 12.07.2023
(73) Proprietor: HITACHI HIGH-TECH CORPORATION, Tokyo 105-6409 (JP)
(72) Inventor: ISHIZUKA, Junji, Tokyo 105-6409 (JP); MITSUYAMA, Toshifumi, Tokyo 105-6409 (JP); ONO, Yukio, Tokyo 105-6409 (JP)
(74) Representative: MERH-IP Matias Erny Reichl Hoffmann Patentanwälte PartG mbB
(86) International application number: PCT/JP2020/033079
(87) International publication number: WO 2022/049626

(56) References cited:
- WO-A1-2012/014367
- JP-A- 2005 333 824
- JP-A- 2006 345 714
- JP-A- 2014 020 779
- JP-A- 2016 202 094
- US-A1- 2005 158 212
- US-A1- 2013 121 881
- US-A1- 2019 302 135

## Description

### Technical Field

The present invention relates to a living body analysis device that analyzes living body substances such as DNA. In particular, the invention relates to a device that performs a pretreatment step for analyzing DNA to be analyzed with a device.

### Background Art

Genomic medicine started fully, and a cancer gene panel diagnostic agent was covered by insurance. A gene profiling test used in cancer gene panel diagnosis is a test in which a nucleic acid is extracted from blood or a pathological specimen collected from a cancer patient, and the nucleic acid is comprehensively analyzed by a next-generation DNA sequencer using a gene mutation test kit reagent.

A treatment policy is determined by a specialist group such as a doctor, a pathologist, a bio-informatician, or a pharmacist based on the analysis result, and then an anticancer agent, a molecular target drug, or an immune checkpoint inhibitor suitable for a patient is provided.

However, since a gene panel test requires a high cost and the number of times that one patient can use the gene panel test in the insurance application is only one, all the tests are required to be successful. Meanwhile, at present, 10 to 20% of specimens are defective as an analysis result.

There are various pretreatment steps for a nucleic acid sample. In the pretreatment step, dispensing of a reagent, amplification of the nucleic acid sample performed by a thermal cycler, purification of DNA, and the like are performed basically by a hand except for some operations. These series of steps have a large number of steps and may have expert knowledge, and there is an increasing need for automation so as not to depend on a skill level of a technician.

A pretreatment automation device that is already commercially available is generally a large device because of units that perform various processes, a gripper for moving a plate between the units, a dispensing machine for dispensing a reagent, and the like.

Meanwhile, in a next-generation sequencer, a large number of DNA fragments to be analyzed, which have been subjected to pretreatment, are fixed on a substrate, and base sequences of the large number of DNA fragments are determined in parallel.

Non-PTL 1 discloses a DNA decoding technique of a next-generation DNA sequencer based on fluorescence detection. In a reaction vessel called a flow cell, clusters in which a plurality of identical DNA fragments are densely packed by an amplification process are disposed at a high density. When a solution containing four types of bases (A, T, G, and C) labeled with four types of phosphors is introduced into the flow cell, complementary bases in the DNA fragments are incorporated by an extension reaction of a polymerase.

Since terminals of the fluorescently labeled bases are modified with a functional group (terminator) for inhibiting the extension reaction, more than one base is not incorporated per DNA fragment. After the extension reaction, excess floating bases are washed away, fluorescence emitted from a reaction spot is detected as a fluorescence spot, and a type of a phosphor is identified by difference in color. After fluorescence detection, terminators and the phosphor on the DNA fragment are dissociated by a chemical reaction to allow a next base to be incorporated.

The extension reaction, fluorescence detection, and terminator dissociation described above are sequentially repeated to decode a sequence of the DNA fragment by about 100 bases.

PTL 1 discloses a method in which a large number of beads on which DNA fragments to be a sample are fixed are disposed in a flow cell, a reagent is supplied to the flow cell, a fluorescence signal generated along with an extension reaction of a base is detected, and a base sequence of the sample is analyzed.

In a pretreatment integrated nucleic acid analysis device, it is necessary to introduce the DNA fragments that have been subjected to a pretreatment step into the flow cell and convey the DNA fragments to an analysis device part that performs a sequence. A work of introduction into the flow cell and conveyance to the device that performs the sequence is currently performed by a hand, and automatic conveyance is also required in accordance with automation of the pretreatment step. Additionally, PTL 2 describes a clinical module for automated sample processing in a laboratory setting. It comprises a robot, centrifuge, and analyzer, along with various carriers and adapters for handling sample tubes. The robot, mounted on a frame with rails and a movable bar, uses a gripper to manipulate tubes, remove caps, and transfer samples between the centrifuge, analyzer, and other stations like a recapping station and storage refrigerator. The system aims to automate tasks like sorting, centrifugation, decapping, analysis, and storage, improving efficiency and reducing manual intervention in laboratory workflows. Eventually, PTL 3 describes a sample pretreatment apparatus featuring a robotic arm with an articulated arm member and a hand. This arm transports sample containers between multiple pretreatment sections, including a dispenser and a centrifuge. The apparatus aims to automate sample handling for different measurements, reducing manual labor and contamination risks.

PTL 3 emphasizes the robotic arm's wide workable range, enabling access to various devices and facilitating the use of generalpurpose laboratory equipment.

The nucleic acid analysis device disclosed in PLT 4 is an automated system configured for sample processing, amplification, and detection, featuring a robotic dispensing unit for liquid handling and a gripper unit for transporting reaction vessels. The apparatus utilizes a central transport mechanism, multiple thermostat baths for thermal processing, and a photometric detection unit with surrounding detectors to analyze samples within specialized reaction vessels.

### Citation List

### Patent Literature

PTL 1: JP2008-528040A
PTL 2: US 2005/158 212 A1
PTL 3: US 2019/302 135 A1
PLT 4: US 2013/121881 A1

### Non-Patent Literature

Non-PTL 1: D. R. Bentley et al., Accurate whole human genome sequencing using reversible terminator chemistry, Nature 456, p.53-59 (2008)

### Summary of Invention

### Technical Problem

In the pretreatment step, it is necessary to perform various processes such as dispensing and mixing of reaction reagents and a temperature control process such as a PCR reaction. In order to automate the pretreatment step, it is necessary to mount a dispensing machine that dispenses the reagents, a temperature control mechanism that performs the PCR reaction or the like, a conveyance mechanism that conveys a plate or the like containing a sample between the mechanisms, a tip used when dispensing the reagents, and the like.

In general, a robot arm that performs an XYZ drive mechanism and a grip mechanism that holds a plate or the like that is a conveyance object are mounted as a conveyance mechanism mounted in a pretreatment automation device.

A gripper that holds the conveyance object is provided below a Z-axis, and a region that can be held by the gripper and a range within which the conveyance object can be conveyed are determined by a stroke of a drive mechanism that drives X- and Y-axes. Therefore, when widening a region accessible by the gripper, it is necessary to enlarge the drive mechanism for XY driving.

In the pretreatment integrated nucleic acid analysis device, the conveyance mechanism conveys an observation object such as the flow cell to a part where an analysis is performed in addition to a range within which a normal pretreatment step is performed, and thus the range to be accessed by the conveyance mechanism becomes wide.

In the pretreatment integrated nucleic acid analysis device, the drive mechanism for the conveyance mechanism is increased in size, and the entire device is increased in size.

Therefore, there is a demand for a technique for widening the accessible range of the conveyance mechanism while limiting the increase in size of the pretreatment integrated nucleic acid analysis device.

An object of the invention is to implement a pretreatment integrated nucleic acid analysis device capable of widening an accessible range of a conveyance mechanism while limiting an increase in size of the device.

### Solution to Problem

In order to achieve the above object, the invention is set out in the appended set of claims. Preferable embodiments are defined in the dependent claims.

A pretreatment mechanism-integrated nucleic acid analysis device includes: an analysis part configured to analyze a specimen; and a pretreatment part configured to perform pretreatment on the specimen and move the pretreated specimen to the analysis part. The pretreatment part includes a dispensing machine configured to dispense a fluid, a holding mechanism configured to hold a member, and a conveyance mechanism configured to move the holding mechanism in a plane direction, an up-down direction, and a rotation direction.

### Advantageous Effects of Invention

According to the invention, it is possible to implement a pretreatment integrated nucleic acid analysis device capable of widening an accessible range of a conveyance mechanism while limiting an increase in size of the device.

Problems, configurations, and effects other than those described above will become apparent from the following description of the embodiments.

### Brief Description of Drawings

[FIG. 1] FIG. 1 is a schematic view showing an outline of a pretreatment mechanism-integrated nucleic acid analysis device according to an embodiment of the invention.
[FIG. 2A] FIG. 2A is a schematic view showing dispensing machine actuators and a gripper mounted in a pretreatment part of the pretreatment integrated nucleic acid analysis device according to the embodiment of the invention.
[FIG. 2B] FIG. 2B is a schematic view showing the dispensing machine actuators and the gripper mounted in the pretreatment part of the pretreatment integrated nucleic acid analysis device according to the embodiment of the invention.
[FIG. 3] FIG. 3 is a schematic view of a conveyance mechanism mounted in the pretreatment mechanism-integrated nucleic acid analysis device according to the present embodiment.
[FIG. 4A] FIG. 4A is a schematic view of a mechanism of the gripper mounted in the pretreatment mechanism-integrated nucleic acid analysis device according to the embodiment of the invention.
[FIG. 4B] FIG. 4B is a schematic view of the mechanism of the gripper mounted in the pretreatment mechanism-integrated nucleic acid analysis device according to the embodiment of the invention.
[FIG. 5] FIG. 5 is a detailed schematic view of the pretreatment part of the pretreatment mechanism-integrated nucleic acid analysis device according to the embodiment of the invention.
[FIG. 6A] FIG. 6A is a diagram illustrating an operation when a plate is conveyed from a reagent mixing unit to a thermal cycler according to the embodiment of the invention.
[FIG. 6B] FIG. 6B is a diagram illustrating the operation when the plate is conveyed from the reagent mixing unit to the thermal cycler according to the embodiment of the invention.
[FIG. 7A] FIG. 7A is a diagram illustrating an operation when conveying an empty tip rack to an empty rack placement location according to the embodiment of the invention.
[FIG. 7B] FIG. 7B is a diagram illustrating the operation when conveying the empty tip rack to the empty rack placement location according to the embodiment of the invention.
[FIG. 8] FIG. 8 is a schematic configuration diagram of a flow cell mounted in the pretreatment mechanism-integrated nucleic acid analysis device according to the embodiment of the invention.
[FIG. 9] FIG. 9 is a diagram illustrating when the flow cell is provided on a stage unit according to the embodiment of the invention.
[FIG. 10] FIG. 10 is a diagram illustrating an operation of the gripper when the flow cell is provided on the stage unit by the conveyance mechanism according to the embodiment of the invention.
[FIG. 11A] FIG. 11A is a schematic plan view of a cooling part.
[FIG. 11B] FIG. 11B is a schematic side view of the cooling part.
[FIG. 12A] FIG. 12A is a schematic plan view of a magnet part.
[FIG. 12B] FIG. 12B is a schematic side view of the magnet part.

### Description of Embodiments

Hereinafter, an embodiment of the invention will be described in detail with reference to the drawings.

Steps, components, and the like for forming components of the embodiment are not limited to the following description. In addition, when a shape and a function of a component are referred to, a component having a shape or a function similar to the shape and arrangement of the component is included unless otherwise specified. In addition, if a main function is not lost even if a part of the components is changed, the components are regarded as equivalent to those before the change.

A pretreatment integrated nucleic acid analysis device according to the invention can perform, by using a flow cell, various analyses such as determination of a DNA sequence. By setting a gene extracted from a pathological part or the like in a device, it is possible to automatically perform a pretreatment necessary for a DNA sequence, introduce a DNA fragment into the flow cell for observation, and perform the DNA sequence by a sequencer.

### Embodiments

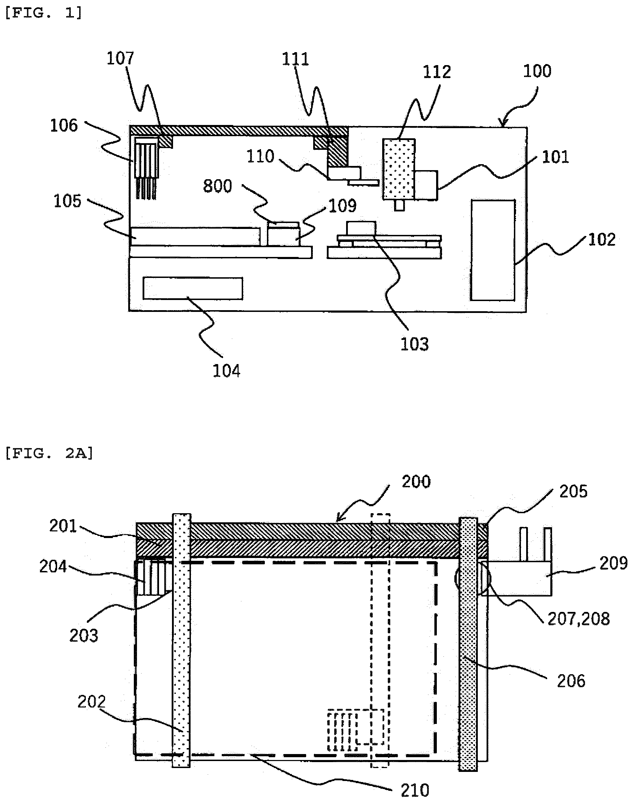

FIG. 1 is a schematic view showing an outline of a pretreatment mechanism-integrated nucleic acid analysis device according to an embodiment of the invention.

In FIG. 1, a pretreatment mechanism-integrated nucleic acid analysis device 100 includes an analysis part including a light source unit 101 that excites a fluorescent dye or the like, a reagent storage unit 102 that stores a reaction reagent, a stage unit 103 that conveys an observation object, and an optical unit 112 that detects fluorescence.

In addition, the pretreatment mechanism-integrated nucleic acid analysis device 100 includes a pretreatment part 200 (shown in FIG. 2A) including a pretreatment mechanism 105 that performs pretreatment on a measurement sample (synonymous with a measurement sample, a living body sample, and a specimen), a dispensing unit 106 that handles a reagent in the pretreatment mechanism 105, a flow cell 800 that loads the measurement sample, a loading unit 109 that introduces a DNA fragment (specimen) for measurement into the flow cell 800, and a gripper unit 110.

The gripper unit 110 performs conveyance in the pretreatment part 200. The dispensing unit 106 is driven by a dispensing mechanism actuator 107, and the gripper unit 110 is driven by a gripper actuator 111.

Operations of the analysis part and the pretreatment part 200 described above are controlled by a control PC 104.

The light source unit 101, the stage unit 103, and the optical unit 112 are provided on a vibration isolation table such that a positional relation between the stage unit 103 and the optical unit 112 is maintained even when there is vibration from an outside.

The reagent storage unit 102 includes a cooling part 1000 (to be described later) using a Peltier element 1103 (to be described later), and can mount a plurality of reagent bottles containing a reagent to be used in the sequence. Although not shown, tubes are connected to the reagent bottles mounted in the reagent storage unit 102. The tubes connected to the reagent bottles are connected to the stage unit 103 by a two-way valve, a three-way valve, a rotary valve, or the like.

The stage unit 103 has an injection opening at a position where the flow cell 800 is mounted, and is connected to an inflow hole of the flow cell 800. An outflow hole of the flow cell 800 is connected to a tube connected to a waste liquid tank. A syringe is provided in a middle of the tube connected to the outflow hole of the flow cell 800, and a reagent mounted in the reagent storage unit 102 flows into the flow cell 800 by operating the syringe to perform various reactions necessary for the sequence.

A living body substance such as DNA to be analyzed, the reagent necessary for the pretreatment, and the flow cell 800 are set in the pretreatment mechanism 105. The living body substance set in the pretreatment mechanism 105 is subjected to a process necessary for analysis using the reagent for pretreatment by the pretreatment part 200.

The pretreated living body substance (specimen) is introduced into the flow cell 800 by using the dispensing unit 106 included in the pretreatment part 200. The flow cell 800 is held by a loading unit (holding part) 109, and the specimen is introduced in this state.

Thereafter, the flow cell 800 is mounted on the stage unit 103 of the analysis part by the gripper unit 110 provided in the pretreatment part 200, and the sequence is performed by the analysis part.

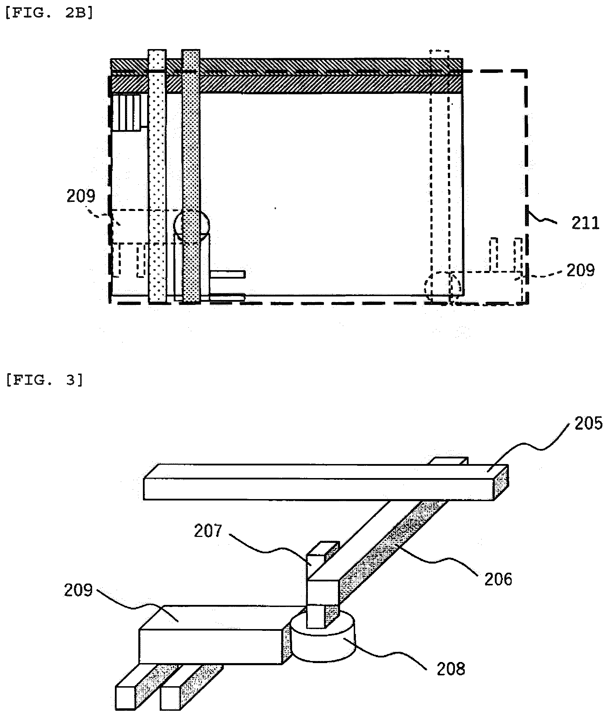

FIGS. 2A and 2B are schematic views showing dispensing machine actuators (201, 202, 203) and a gripper (holding mechanism) 209 mounted in the pretreatment part 200 of the pretreatment integrated nucleic acid analysis device according to the embodiment of the invention, and show an upper surface of the pretreatment part 200.

In FIG. 2A, the pretreatment part 200 includes a dispensing machine 204 that handles (dispenses) a fluid such as a specimen or a reagent, the dispensing machine X-axis actuator 201 that drives the dispensing machine 204, the dispensing machine Y-axis actuator 202, the dispensing machine Z-axis actuator 203, the gripper 209 that conveys a plate, a tip rack, and the flow cell 800 to be described later, a gripper X-axis actuator 205 that drives the gripper 209, a gripper Y-axis actuator 206, a gripper Z-axis actuator 207, and a gripper θ-axis actuator (θ-axis movement mechanism (holding mechanism rotation actuator)) 208.

The gripper 209 is moved in a plane direction by the gripper X-axis actuator 205 and the gripper Y-axis actuator 206, and the gripper 209 is moved in an up-down direction by the gripper Z-axis actuator 207. In addition, the gripper 209 is moved in a rotation direction by the gripper θ-axis actuator 208.

A plurality of dispensing machines 204 are mounted in order to enable simultaneous processing of a plurality of specimens, and dispensing operation can be controlled independently of each other. Each of the dispensing machines 204 may be, for example, a pneumatic electric pipetter driven by a cylinder. Although not shown, a dispensing mechanism necessary for dispensing operations (an aspiration operation and a discharge operation) is incorporated in the dispensing machine 204, and the dispensing mechanism includes a cylinder in which a plunger is incorporated, a motor that reciprocates the plunger, and the like.

In addition, a plurality of mounted dispensing machines 204 are arranged side by side, and are arranged such that the aspiration and discharge operations can be performed in accordance with a plate or a tube having standard dimensions defined by American Standards Association (ANSI) and Biomolecular Chemical Standards Association (SBS).

In addition, each of the dispensing machines 204 includes, for example, a capacitive liquid level detection unit, and feeds back detection of a liquid level to control of the dispensing machine Z-axis actuator 203 during a reagent aspiration operation. The dispensing machine 204 lowers in a Z-axis direction by a specified amount after the liquid level detection, and performs the reagent aspiration operation. The dispensing machine Z-axis actuator 203 is driven in conjunction with the aspiration operation of the dispensing machine 204.

By the operation, it is possible to aspirate a specified amount of reagent even when a reagent amount of an aspiration destination is small. At the time of discharging the reagent, the liquid level detection is performed in the same manner as that at the time of aspirating the reagent, and then a Z-axis is raised or lowered in accordance with the liquid to be discharged, and the discharge operation is performed. The operation prevents air bubbles from being mixed into the discharged solution.

Each of the dispensing machine XYZ-axis actuators 201, 202, and 203 may be, for example, an electric actuator driven by a belt. Although not shown in the drawings, the actuator of each axis includes a motor or the like necessary for driving. In addition, a PI sensor and an encoder that checks a movement accuracy are provided such that an origin of each axis can be detected.

The axes for driving the dispensing machine 204 and the gripper 209 have different origins. For example, as shown in FIG. 2A, the origin of the dispensing machine 204 is on an upper left side, and the origin of the gripper 209 is on an upper right side. In addition, positions of the dispensing machine 204 and the gripper 209 are constantly monitored, and are controlled so as not to collide with each other.

In the pretreatment mechanism-integrated nucleic acid analysis device 100 according to the present embodiment, a disposable tip is used to prevent contamination between samples. The dispensing machine 204 moves to a position where the tip racks are stored by the dispensing machine X-axis actuator 201 and the dispensing machine Y-axis actuator 202. Thereafter, the dispensing machine Z-axis actuator 203 is driven to be lowered to equip a tip. After the tip is equipped, the dispensing machine Z-axis actuator 203 is driven to be raised to a position of a height that does not interfere with other units, and moves to a reagent aspiration position of a reagent storage part or the like by the dispensing machine X-axis actuator 201 and the dispensing machine Y-axis actuator 202.

The dispensing machine 204 drives in a range indicated by a dispensing machine operation range 210 shown in FIG. 2A, and performs operations such as attachment and detachment of a tip and aspiration and discharge of a reagent. The dispensing machine operation range 210 is located in the upper surface of the pretreatment part 200.

FIG. 3 is a schematic view of a conveyance mechanism mounted on the pretreatment mechanism-integrated nucleic acid analysis device 100 according to the present embodiment. The conveyance mechanism includes the gripper X-axis actuator 205, the gripper Y-axis actuator 206, the gripper Z-axis actuator 207, and the gripper θ-axis actuator 208.

In FIG. 3, the gripper θ-axis actuator 208 for the gripper 209 is supported by the Z-axis extending in the Z-axis direction of the gripper Z-axis actuator 207, and is disposed coaxially. The gripper 209 performing a holding operation is disposed on a plane orthogonal to axes of the gripper Z-axis actuator 207 and the gripper θ-axis actuator 208 and parallel to a driving surface driven by the gripper X-axis actuator 205 and the gripper Y-axis actuator 206.

The gripper 209 is moved to a specified position by the gripper X-axis actuator 205 and the gripper Y-axis actuator 206, and then rotatably moved by the gripper θ-axis actuator 208 in accordance with an orientation of a holding object. Thereafter, the gripper 209 is lowered to a holding position by the gripper Z-axis actuator 207, and holds the holding object.

Similarly at a movement destination, the gripper 209 rotates in accordance with a provision orientation of the holding object. By a planar driving performed by the gripper X-axis actuator 205 and the gripper Y-axis actuator 206 and the rotational driving performed by the gripper θ-axis actuator 208, a conveyance operation can be performed within the range indicated by a gripper operation range 211 shown in FIG. 2B. The gripper operation range 211 is a range wider than the dispensing machine operation range 210. That is, a region in which the gripper 209 that is the holding mechanism moves is larger than movement ranges of the gripper X-axis actuator 205 that is an X-axis direction movement mechanism and the gripper Y-axis actuator 206 that is a Y-axis direction movement mechanism, and can access an outside of an access region of the dispensing machine 204.

This is because the gripper 209 can be rotationally driven and thus can move to a region beyond the dispensing machine operation range 210.

In the pretreatment mechanism-integrated nucleic acid analysis device 100 according to the present embodiment, the conveyance mechanism conveys the tip rack containing a tip for a dispensing machine, a well plate for mixing reagents, and the flow cell that introduces the pretreated living body substance.

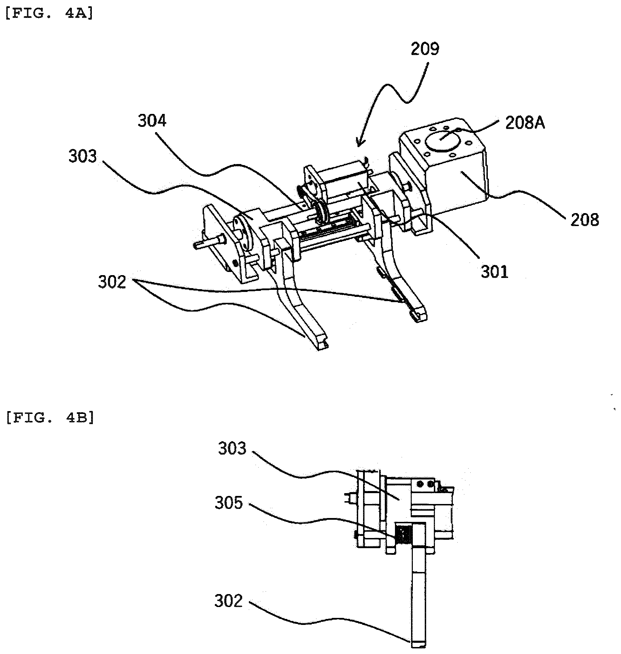

FIGS. 4A and 4B are schematic views of the mechanism of the gripper 209 mounted in the pretreatment mechanism-integrated nucleic acid analysis device 100 according to the present embodiment.

In FIGS. 4A and 4B, the gripper 209 includes a grip arm 302 that holds the plate, the flow cell, or the like, a grip arm actuator 301 that drives the grip arm 302, translation parts 303 that move in conjunction with the grip arm actuator 301, an elastic body 305 that connects the translation parts 303 and the grip arm 302, and a screw mechanism 304 that moves the translation parts 303 by the grip arm actuator 301.

The gripper θ-axis actuator 208 includes a θ-axis rotation shaft 208A that rotates the gripper 209 to change an orientation. The grip arm 302 has two arms facing each other. The translation parts 303 are members that drive the two arms of the grip arm 302 simultaneously. An extension line of a straight line connecting the two arms of the grip arm 302 to each other is orthogonal to the Z-axis direction.

The screw mechanism 304 is configured such that an orientation of a screw thread is reversed in a central part, and the translation parts 303 disposed on both sides of the screw mechanism 304 can be driven by one grip arm actuator 301. The grip arm 302 is coupled to the translation parts 303 by the elastic body 305, and is driven in accordance with the translation parts 303.

After the gripper 209 is moved to an upper part of a holding object by the gripper X-axis actuator 205, the gripper Y-axis actuator 206, and the gripper θ-axis actuator 208, the screw mechanism 304 is driven by the grip arm actuator 301. Driving the screw mechanism 304 increases an interval between the translation parts 303. When the translation parts 303 move, the translation parts 303 move in a direction in which an interval between the grip arm 302 increases through the elastic body 305.

After the interval between the grip arm 302 is widened to a specified width, the gripper Z-axis actuator 207 is driven to be lowered to a position where the grip arm 302 reaches a side surface of the holding object. Thereafter, the screw mechanism 304 is reversely rotated by the grip arm actuator 301, and is driven in a direction in which the interval between the two arms of the grip arm 302 is narrowed. The grip arm actuator 301 holds the object by adjusting a driving amount of the screw mechanism 304 in accordance with a width of the object in a holding direction.

After the gripper 209 holds the holding object, the gripper Z-axis actuator 207 is driven to be raised to a position where the gripper Z-axis actuator 207 does not interfere with other units, and moves to a conveyance destination by the gripper X-axis actuator 205, the gripper Y-axis actuator 206, and the gripper θ-axis actuator 208.

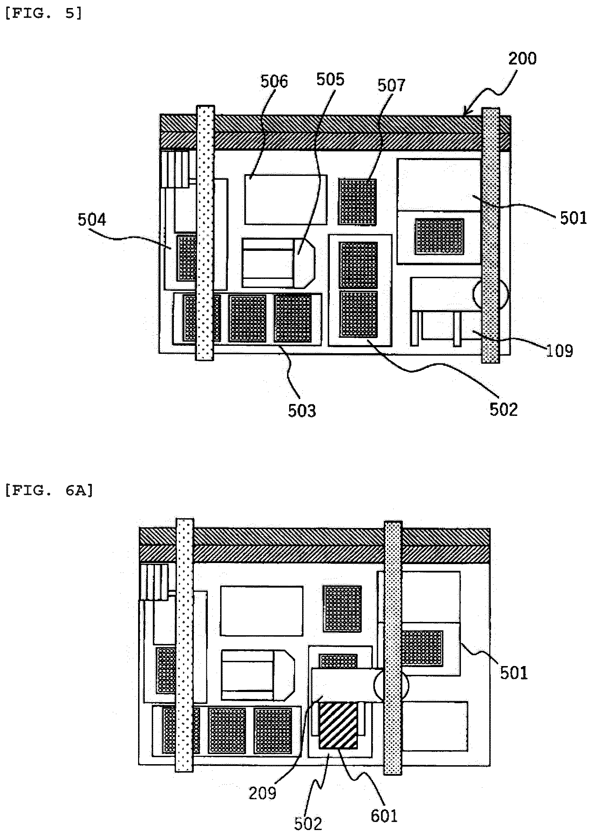

FIG. 5 is a detailed schematic view of the pretreatment part 200 of the pretreatment mechanism-integrated nucleic acid analysis device 100 according to the present embodiment. FIG. 5 shows the pretreatment part 200 in more detail than FIGS. 2A and 2B.

In FIG. 5, the pretreatment part 200 includes a thermal cycler (temperature control mechanism) 501 that performs heating and cooling in order to perform a temperature control reaction such as a PCR reaction, a loading unit 109 that loads a measurement sample, for which pretreatment is completed, in the flow cell 800, a reagent mixing unit 502 that mixes a reagent and the measurement sample, a tip rack 503 that holds a tip used for dispensing, a reagent storage unit 504 that stores the reagent used for the pretreatment, a purification unit 505 that performs a purification step, a detection unit 506 that performs concentration quantification after the purification, and magnet plates (magnet parts) 507 that collect a magnetic bead to which a specimens is fixed.

The reagent mixing unit 502 and the reagent storage unit 504 include a Peltier element 1103 to be described later, a heat sink 1104 for air cooling, an air cooling fan 1101, and a cooling block 1102, and are temperature-controlled to a constant temperature.

The cooling block 1102 is a block in which holes by which a plate or a tube having a standard dimension defined by American Society of Standards (ANSI) and Society of Biomolecular Chemical Standards (SBS) can be provided are formed. Although not shown, the tip rack 503 is disposed on a plane that can be driven in the up-down direction, and a plurality of tips are stacked in a vertical direction.

The reagent for pretreatment is provided on the reagent storage unit 504 and dispensed to a plate provided on the reagent mixing unit 502 by the dispensing machine 204. The plate into which the reagent is dispensed is stirred and mixed by the dispensing machine 204 or a shaker (not shown).

After the stirring, the plate is held by the gripper 209 and conveyed to the thermal cycler 501. Similarly to the reagent mixing unit 502 and the reagent storage unit 504, the thermal cycler 501 also includes a block having a groove conforming to a shape of a bottom surface of the plate.

The thermal cycler 501 includes, for example, a sliding lid driven by a ball screw. After the plate is moved to the thermal cycler 501, a lid for preventing evaporation is placed as necessary, and the lid of the thermal cycler 501 is closed. A heater is incorporated in the lid of the thermal cycler 501, and an upper surface of the plate is brought into a high temperature state while a temperature control reaction such as PCR is performed, thereby preventing a decrease in reaction efficiency and a decrease in the amount of reaction liquid due to evaporation or the like.

The plate generally has a rectangular shape. In addition, since the reagent mixing unit 502 and the thermal cycler 501 mounted on the pretreatment part 200 have a block conforming to the shape of the plate, each component is generally disposed such that the plate is provided in the same direction.

In the present embodiment, since the drive mechanism of the gripper 209 has a rotation shaft, it is not necessary to align plate provision directions of parts at a movement destination of the plate.

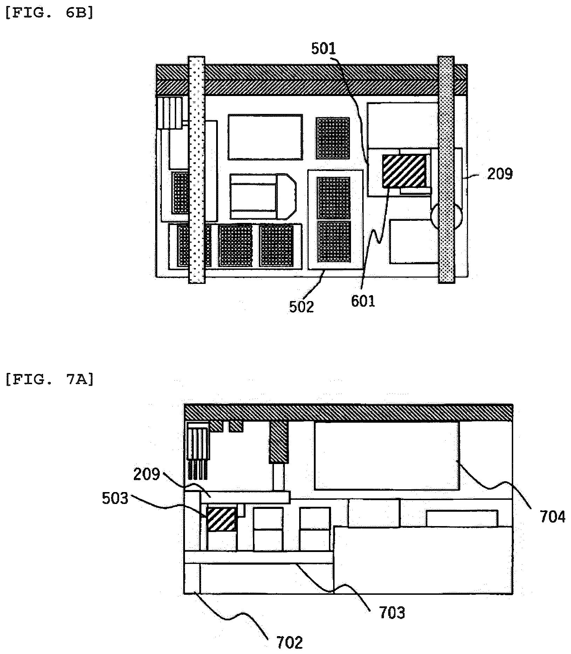

FIGS. 6A and 6B are diagrams for illustrating an operation when the plate is conveyed from the reagent mixing unit 502 to the thermal cycler 501 according to the present embodiment.

As shown in FIGS. 6A and 6B, in the pretreatment mechanism-integrated nucleic acid analysis device 100 according to the present embodiment, provision orientations of plates at the reagent mixing unit 502 and the thermal cycler 501 are not aligned. As shown in FIG. 6A, a mixing plate 601 provided on the reagent mixing unit 502 in which the dispensing of the reagent is completed is held by the gripper 209.

Thereafter, as shown in FIG. 6B, the gripper 209 is moved to the conveyance destination by the gripper X-axis actuator 205 and the gripper Y-axis actuator 206, and an orientation of the gripper 209 is rotated by 90° by the gripper θ-axis actuator 208.

Because of the operation, it is not necessary to align the provision directions of the plates between units that performs conveyance, and it is possible to provide the units in a space-saving manner in accordance with a shape of each part.

The pretreatment mechanism-integrated nucleic acid analysis device 100 according to the invention uses disposable tips. The tips are arranged in eight rows and twelve columns in the tip rack 503, and 96 tips are contained in one tip rack 503. In the pretreatment step, it is necessary to dispense and mix various reagents. Therefore, in order to complete a pretreatment step once, tips corresponding to a plurality of tip racks 503 are required.

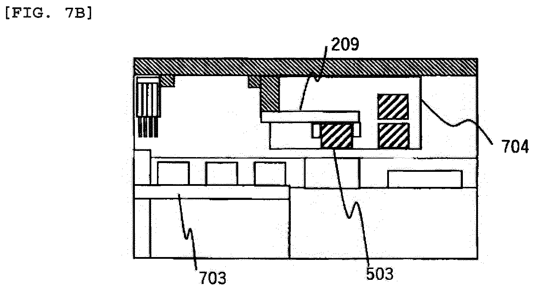

FIGS. 7A and 7B are diagrams illustrating an operation when conveying the empty tip rack 503 to an empty rack placement location 704 according to the present embodiment.

In FIGS. 7A and 7B, in the pretreatment mechanism-integrated nucleic acid analysis device 100 according to the present embodiment, examples of a used tip rack placement location includes the empty rack placement location 704 below the dispensing machine X-axis actuator 201 and the gripper X-axis actuator 205.

On a tip rack table 703 of the pretreatment mechanism-integrated nucleic acid analysis device 100, the plurality of tip racks 503 are disposed in a vertically stacked state. The empty tip rack 503 using all the tips is held by the gripper 209. Thereafter, the gripper X-axis actuator 205 and the gripper Y-axis actuator 206 move the gripper 209 to the vicinity of the empty rack placement location 704, and the gripper θ-axis actuator 208 rotates the orientation of the gripper 209 by 180°. After the rotation by 180°, the gripper X-axis actuator 205 and the gripper Y-axis actuator 206 are driven again, and the empty tip rack 503 is moved to the empty rack placement location 704 disposed on a wall side of the pretreatment mechanism-integrated nucleic acid analysis device 100.

Then, a state shown in FIG. 7B is obtained. The tip rack 503 disposed on the tip rack table 703 is moved by a tip rack actuator 702.

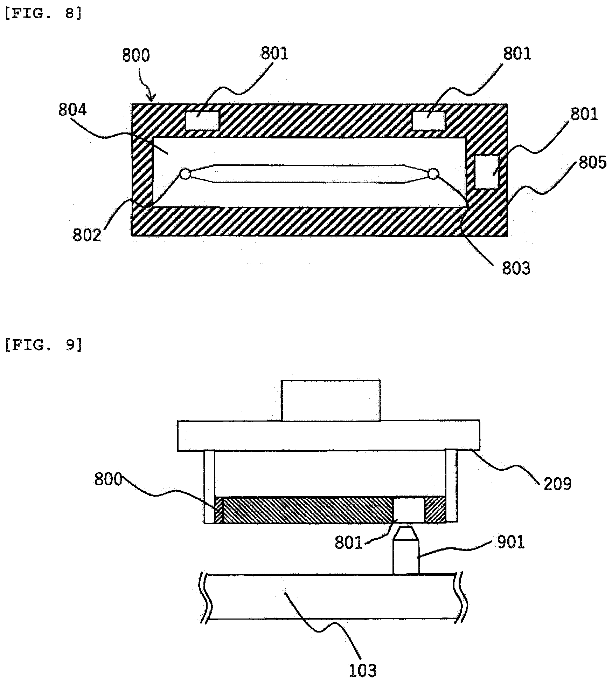

FIG. 8 is a schematic configuration diagram of the flow cell 800 mounted in the pretreatment mechanism-integrated nucleic acid analysis device 100 according to the embodiment of the invention.

In FIG. 8, the flow cell 800 includes a glass substrate 804 including two glass plates and an intermediate member, and a flow cell case 805 having positioning holes. A central part of the intermediate member is hollowed out. In addition, an inflow hole 802 and a discharge hole 803 are formed in one glass plate of the glass substrate 804, and the inflow hole 802 and the discharge hole 803 are connected to the hollowedout part of the intermediate member, thereby forming a flow path configuration through which a liquid can pass.

The flow cell case 805 includes a plurality of positioning holes 801 into which positioning pins are inserted. Meanwhile, the loading unit 109 and the stage unit 103 include the positioning pins corresponding to the positioning holes 801 formed in the flow cell case 805.

FIG. 9 is a diagram illustrating when the flow cell 800 is provided on the stage unit 103 according to the embodiment of the invention.

In FIG. 9, the flow cell 800 held by the gripper 209 is conveyed to the stage unit 103. The gripper X-axis actuator 205, the gripper Y-axis actuator 206, and the gripper θ-axis actuator 208 are driven such that the positioning hole 801 is located above a positioning pin 901. Further, the gripper Z-axis actuator 207 is driven to be lowered such that the positioning pin 901 is inserted into the positioning hole 801.

In the pretreatment mechanism-integrated nucleic acid analysis device 100 according to the present embodiment, it is necessary to accurately align a position of the inflow hole 802 of the flow cell with respect to the injection opening of the loading unit 109 and the provision position of the glass substrate 804 with respect to the stage unit 103.

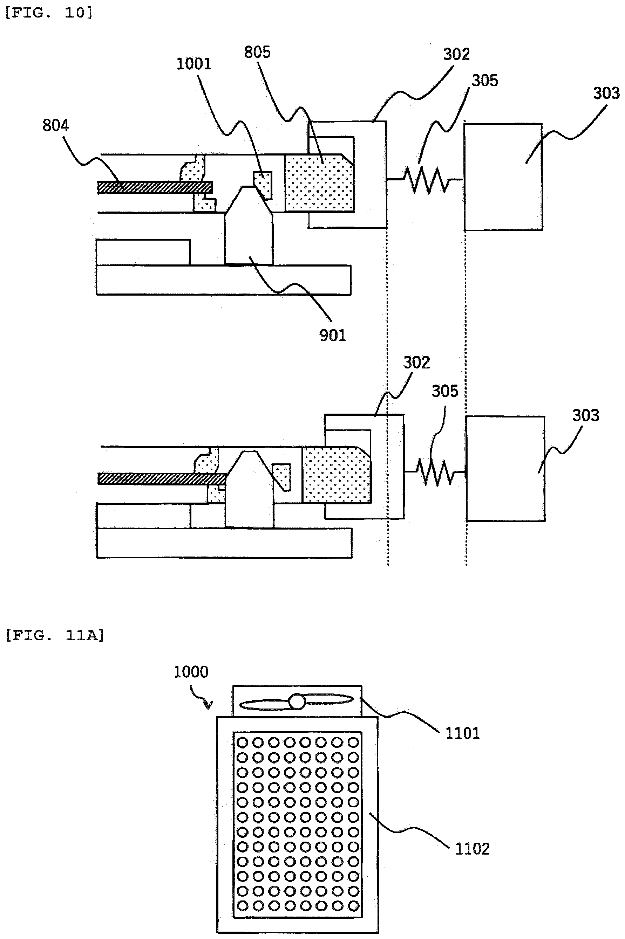

FIG. 10 is a diagram illustrating an operation of the gripper 209 when the flow cell 800 is provided on the stage unit 103 by the conveyance mechanism according to the embodiment of the invention.

In FIG. 10, the positioning pin 901 includes a taper, and the flow cell case 805 includes a guide member 1001. As the gripper 209 is lowered, a tapered part of the positioning pin 901 comes into contact with the guide member 1001. Since the positioning pin 901 includes the taper, the flow cell 800 is provided along the taper.

In a case where there is a deviation in the plane direction when the flow cell 800 is provided on the stage unit 103, the flow cell case 805 moves along the taper of the positioning pin 901. In the case of FIG. 10, the flow cell case 805 slides rightward. The grip arm 302 moves in accordance with the movement of the flow cell case 805. The elastic body 305 between the grip arm 302 and the translation part 303 is deformed to correct positional deviation at the time of insertion into the positioning pin 901.

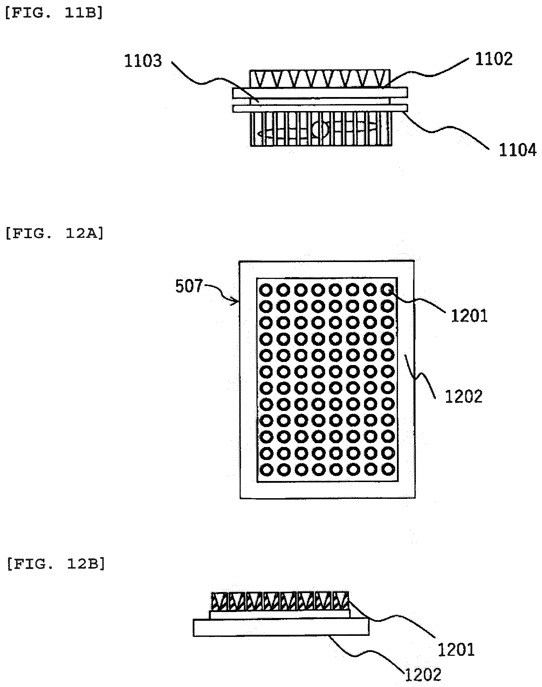

FIG. 11A is a schematic plan view of the cooling part 1000, and FIG. 11B is a schematic side view of the cooling part 1000.

In FIGS. 11A and 11B, the cooling part 1000 includes the air cooling fan 1101, the cooling block 1102, the Peltier element 1103, and the heat sink 1104. The Peltier element 1103 is disposed between the cooling block 1102 and the heat sink 1104.

FIG. 12A is a schematic plan view of the magnet part 507, and FIG. 12B is a schematic side view of the magnet part 507.

In FIGS. 12A and 12B, the magnet part 507 includes magnets 1201 and a magnet base 1202. The magnets 1201 are disposed on the magnet base 1202.

As described above, according to the embodiment of the invention, the pretreatment mechanism-integrated nucleic acid analysis device 100 includes the gripper X-axis actuator 205 that moves the gripper 209 that is the conveyance mechanism that conveys the flow cell 800 and the like in an X-axis direction, the gripper Y-axis actuator 206 that moves the gripper 209 in a Y-axis direction, the gripper Z-axis actuator 207 that moves the gripper 209 in the Z-axis direction, and the gripper θ-axis actuator 208 that moves the gripper 209 in a θ-axis direction.

With the above configuration, the gripper 209 is movable not only in the X-axis direction, the Y-axis direction, and the Z-axis direction, but also in the θ-axis direction (rotation direction), and a movement range of the gripper 209 that is the conveyance mechanism can be expanded. The conveyance mechanism (the gripper X-axis actuator 205, the gripper Y-axis actuator 206, the gripper Z-axis actuator 207, the gripper θ-axis actuator 208, and the gripper 209) can be reduced in size while securing a necessary movement range of the flow cell or the like by the pretreatment part 200. That is, it is possible to implement a pretreatment integrated nucleic acid analysis device capable of widening the accessible range of the conveyance mechanism while limiting an increase in size of the device.

### Reference Signs List

- 100:: pretreatment mechanism-integrated nucleic acid analysis device
- 101:: light source unit
- 102:: reagent storage unit
- 103:: stage unit
- 104:: control PC
- 105:: pretreatment mechanism
- 106:: dispensing unit
- 107:: dispensing machine actuator
- 109:: loading unit
- 110:: gripper unit
- 111:: gripper actuator
- 112:: optical unit
- 200:: pretreatment part
- 201:: dispensing machine X-axis actuator
- 202:: dispensing machine Y-axis actuator
- 203:: dispensing machine Z-axis actuator
- 204:: dispensing machine
- 205:: gripper X-axis actuator
- 206:: gripper Y-axis actuator
- 207:: gripper Z-axis actuator
- 208:: gripper θ-axis actuator
- 209:: gripper
- 210:: dispensing machine operation range
- 211:: gripper operation range
- 301:: grip arm actuator
- 302:: grip arm
- 303:: translation member
- 304:: screw mechanism
- 305:: elastic body
- 501:: thermal cycler (temperature control mechanism)
- 502:: reagent mixing unit
- 503:: tip rack
- 504:: reagent storage unit
- 505:: purification unit
- 506:: detection unit
- 507:: magnet plate (magnet part)
- 601:: mixing plate
- 702:: tip rack actuator
- 703:: tip rack table
- 704:: empty rack placement location
- 800:: flow cell
- 801:: positioning hole
- 802:: inflow hole
- 803:: discharge hole
- 804:: glass substrate
- 805:: flow cell case
- 901:: positioning pin
- 1000:: cooling part
- 1001:: guide member
- 1101:: air cooling fan
- 1102:: cooling block
- 1103:: Peltier element
- 1104:: heat sink
- 1201:: magnet
- 1202:: magnet base

## Claims

1. A pretreatment mechanism-integrated nucleic acid analysis device (100), comprising:
an analysis part configured to analyze a specimen; and
a pretreatment part (200) configured to perform pretreatment on the specimen and move the pretreated specimen to the analysis part, wherein
the pretreatment part (200) includes
a dispensing machine (204) configured to dispense a fluid,
a holding mechanism (209) configured to hold a member, and
a conveyance mechanism configured to move the holding mechanism (209) in a plane direction, an up-down direction, and a rotation direction, wherein
the conveyance mechanism includes an X-axis direction movement mechanism (205), a Y-axis direction movement mechanism (206), a Z-axis direction movement mechanism (207), and a θ-axis movement mechanism (208), and the holding mechanism (209) is moved in the plane direction by the X-axis direction movement mechanism and the Y-axis direction movement mechanism (206), moved in the up-down direction by the Z-axis direction movement mechanism (207), and moved in the rotation direction by the θ-axis movement mechanism (208), wherein
the θ-axis movement mechanism (208) is supported by a Z-axis extending in a Z-axis direction of the Z-axis direction movement mechanism (207), wherein
the pretreatment part (200) further comprises a dispensing machine X-axis actuator (201) and a dispensing machine Y-axis actuator (202), the actuators being adapted to move the dispensing machine (204) to different positions, wherein
a region in which the holding mechanism (209) moves is larger than a movement range of the X-axis direction movement mechanism and the Y-axis direction movement mechanism (206), and is able to access an outside of an access region of the dispensing machine (204), and wherein
the access region is defined by the points being reachable through movements caused by the dispensing machine X-axis actuator (201) and the dispensing machine Y-axis actuator (202).

2. The pretreatment mechanism-integrated nucleic acid analysis device (100) according to claim 1, wherein
the θ-axis movement mechanism (208) is a holding mechanism rotation actuator that rotates the holding mechanism (209), and
the holding mechanism (209) includes
a grip arm (302) configured to grip the member and having two arms facing each other,
a grip arm actuator (301) configured to drive the two arms of the grip arm (302),
a translation part (303) configured to move in conjunction with the grip arm actuator (301) and drive the two arms simultaneously, and
an elastic body (305) connecting the translation part (303) and the two arms.

3. The pretreatment mechanism-integrated nucleic acid analysis device (100) according to claim 2, wherein
an extension line of a straight line connecting the two arms to each other is orthogonal to the Z-axis direction.

4. The pretreatment mechanism-integrated nucleic acid analysis device (100) according to claim 1, wherein
the pretreatment part (200) includes
a temperature control mechanism (501) configured to perform heating and cooling in order to perform a temperature control reaction,
a magnet part (507) configured to collect a magnetic bead to which the specimen is fixed,
a flow cell (800) into which the specimen is introduced, and
a holding part (109) configured to hold the flow cell (800) in order to introduce the specimen into the flow cell (800).

5. The pretreatment mechanism-integrated nucleic acid analysis device (100) according to claim 4, wherein
the analysis part includes
a light source unit (101) and an optical unit (112) configured to optically detect the specimen in the flow cell (800), and
a stage unit (103) configured to mount and convey the flow cell (800).

## Patentansprüche

1. Nucleinsäure-Analysevorrichtung (100) mit integriertem Vorbehandlungsmechanismus, die Folgendes umfasst:
eine Analysekomponente, die konfiguriert ist, eine Probe zu analysieren; und
eine Vorbehandlungskomponente (200), die konfiguriert ist, eine Vorbehandlung an der Probe durchzuführen und die vorbehandelte Probe zur Analysekomponente zu bewegen, wobei
die Vorbehandlungskomponente (200) Folgendes enthält:
eine Ausgabemaschine (204), die konfiguriert ist, ein Fluid abzugeben,
einen Haltemechanismus (209), der konfiguriert ist, ein Element zu halten, und
einen Fördermechanismus, der konfiguriert ist, den Haltemechanismus (209) in einer Ebenenrichtung, einer Oben-Unten-Richtung und einer Drehrichtung zu bewegen, wobei
der Fördermechanismus einen X-Achsenrichtungs-Bewegungsmechanismus (205), einen Y-Achsenrichtungs-Bewegungsmechanismus (206), einen Z-Achsenrichtungs-Bewegungsmechanismus (207) und einen θ-Achsen-Bewegungsmechanismus (208) enthält und der Haltemechanismus (209) durch den X-Achsenrichtungs-Bewegungsmechanismus und den Y-Achsenrichtungs-Bewegungsmechanismus (206) in der Ebenenrichtung bewegt wird, durch den Z-Achsenrichtungs-Bewegungsmechanismus (207) in der Oben-Unten-Richtung bewegt wird und durch den θ-Achsen-Bewegungsmechanismus (208) in der Drehrichtung bewegt wird, wobei
der θ-Achsen-Bewegungsmechanismus (208) durch eine Z-Achse getragen wird, die sich in einer Z-Achsenrichtung des Z-Achsenrichtungs-Bewegungsmechanismus (207) erstreckt, wobei
die Vorbehandlungskomponente (200) ferner einen Ausgabemaschinen-X-Achsenaktor (201) und einen Ausgabemaschinen-Y-Achsenaktor (202) umfasst, wobei die Aktoren ausgelegt sind, die Ausgabemaschine (204) an verschiedene Positionen zu bewegen, wobei
ein Bereich, in dem sich der Haltemechanismus (209) bewegt, größer als ein Bewegungsbereich des X-Achsenrichtungs-Bewegungsmechanismus und des Y-Achsen-Bewegungsmechanismus (206) ist, und auf außerhalb eines Zugriffsbereichs der Ausgabemaschine (204) zugreifen kann, und wobei
der Zugriffsbereich durch die Punkte definiert ist, die durch Bewegungen erreichbar sind, die durch den Ausgabemaschinen-X-Achsenaktor (201) und den Ausgabemaschinen-Y-Achsenaktor (202) bewirkt werden.

2. Nucleinsäure-Analysevorrichtung (100) mit integriertem Vorbehandlungsmechanismus nach Anspruch 1, wobei
der θ-Achsen-Bewegungsmechanismus (208) ein Haltemechanismus-Drehaktor ist, der den Haltemechanismus (209) dreht, und
der Haltemechanismus (209) Folgendes enthält:
einen Greifarm (302), der konfiguriert ist, das Element zu greifen, und zwei Arme aufweist, die einander zugewandt sind,
einen Greifarmaktor (301), der konfiguriert ist, die zwei Arme des Greifarms (302) anzutreiben,
eine Translationskomponente (303), die konfiguriert ist, sich in Verbindung mit dem Greifarmaktor (301) zu bewegen und die zwei Arme gleichzeitig anzutreiben, und
einen elastischen Körper (305), der die Translationskomponente (303) und die zwei Arme verbindet.

3. Nucleinsäure-Analysevorrichtung (100) mit integriertem Vorbehandlungsmechanismus nach Anspruch 2, wobei
eine Erstreckungslinie einer geraden Linie, die die zwei Arme miteinander verbindet, zur Z-Achsenrichtung senkrecht ist.

4. Nucleinsäure-Analysevorrichtung (100) mit integriertem Vorbehandlungsmechanismus nach Anspruch 1, wobei
die Vorbehandlungskomponente (200) Folgendes enthält:
einen Temperatursteuermechanismus (501), der konfiguriert ist, ein Erwärmen und Kühlen durchzuführen, um eine Reaktion mit Temperatursteuerung durchzuführen,
eine Magnetkomponente (507), die konfiguriert ist, eine magnetische Perle, an der die Probe befestigt ist, zu erfassen,
eine Durchflusszelle (800), in die die Probe eingeleitet wird, und
eine Haltekomponente (109), die konfiguriert ist, die Durchflusszelle (800) zu halten, um die Probe in die Durchflusszelle (800) einzuleiten.

5. Nucleinsäure-Analysevorrichtung (100) mit integriertem Vorbehandlungsmechanismus nach Anspruch 4, wobei
die Analysekomponente Folgendes enthält:
eine Lichtquelleneinheit (101) und eine optische Einheit (112), die konfiguriert ist, die Probe in der Durchflusszelle (800) optisch zu detektieren, und
eine Bühneneinheit (103), die konfiguriert ist, die Durchflusszelle (800) aufzunehmen und zu befördern.

## Revendications

1. Dispositif d'analyse d'acide nucléique à mécanisme de prétraitement intégré (100), comprenant :
une partie d'analyse configurée pour analyser un prélèvement ; et
une partie de prétraitement (200) configurée pour effectuer un prétraitement sur le prélèvement et déplacer le prélèvement prétraité jusqu'à la partie d'analyse, dans lequel
la partie de prétraitement (200) inclut
une machine de distribution (204) configurée pour distribuer un fluide,
un mécanisme de maintien (209) configurée pour maintenir un élément, et
un mécanisme de convoyage configuré pour déplacer le mécanisme de maintien (209) dans une direction plane, une direction haut/bas, et une direction de rotation, dans lequel
le mécanisme de convoyage inclut un mécanisme de déplacement dans une direction selon l'axe X (205), un mécanisme de déplacement dans une direction selon l'axe Y (206), un mécanisme de déplacement dans une direction selon l'axe Z (207), et un mécanisme de déplacement selon l'axe θ (208), et le mécanisme de maintien (209) est déplacé dans la direction plane par le mécanisme de déplacement dans une direction selon l'axe X et le mécanisme de déplacement dans une direction selon l'axe Y, déplacé dans la direction haut/bas par le mécanisme de déplacement dans une direction selon l'axe Z (207), et déplacé dans la direction de rotation par le mécanisme de déplacement selon l'axe θ (208), dans lequel
le mécanisme de déplacement selon l'axe θ (208) est supporté par un axe Z s'étendant dans une direction selon l'axe Z du mécanisme de déplacement dans une direction selon l'axe Z (207), dans lequel
la partie de prétraitement (200) comprend en outre un actionneur selon l'axe X de machine de distribution (201) et un actionneur selon l'axe Y de machine de distribution (202), les actionneurs étant adaptés pour déplacer la machine de distribution (204) à des positions différentes, dans lequel
une région dans laquelle le mécanisme de maintien (209) se déplace est plus grande qu'une plage de déplacement du mécanisme de déplacement dans une direction selon l'axe X et du mécanisme de déplacement dans une direction selon l'axe Y (206), et est apte à accéder à l'extérieur d'une région d'accès de la machine de distribution (204), et dans lequel
la région d'accès est définie par les points qui peuvent être atteints par des déplacements entraînés par l'actionneur selon l'axe X de machine de distribution (201) et l'actionneur selon l'axe Y de machine de distribution (202).

2. Dispositif d'analyse d'acide nucléique à mécanisme de prétraitement intégré (100) selon la revendication 1, dans lequel
le mécanisme de déplacement selon l'axe θ (208) est un actionneur de rotation de mécanisme de maintien qui met en rotation le mécanisme de maintien (209), et
le mécanisme de maintien (209) inclut
un bras de préhension (302) configuré pour saisir l'élément et ayant deux bras se faisant face l'un et l'autre,
un actionneur de bras de préhension (301) configuré pour entraîner les deux bras du bras de préhension (302),
une partie de translation (303) configurée pour se déplacer conjointement avec l'actionneur de bras de préhension (301) et pour entraîner les deux bras simultanément, et
un corps élastique (305) connectant la partie de translation (303) et les deux bras.

3. Dispositif d'analyse d'acide nucléique à mécanisme de prétraitement intégré (100) selon la revendication 2, dans lequel
une ligne de prolongement d'une ligne droite relint les deux bras l'un à l'autre est orthogonale à la direction selon l'axe Z.

4. Dispositif d'analyse d'acide nucléique à mécanisme de prétraitement intégré (100) selon la revendication 1, dans lequel
la partie de prétraitement (200) inclut
un mécanisme de commande de température (501) configuré pour effectuer un chauffage et un refroidissement afin d'effectuer une réaction de commande de température,
une partie aimantée (57) configurée pour collecter une bille magnétique à laquelle le prélèvement est fixé,
une cellule d'écoulement (800) dans laquelle le prélèvement est introduit, et une partie de maintien (109) configurée pour maintenir la cellule d'écoulement (800) afin d'introduire le prélèvement dans la cellule d'écoulement (800).

5. Dispositif d'analyse d'acide nucléique à mécanisme de prétraitement intégré (100) selon la revendication 4, dans lequel
la partie d'analyse inclut
une unité à source lumineuse (101) et une unité optique (112) configurée pour détecter optiquement le prélèvement dans la cellule d'écoulement (800), et
une unité d'étage (103) configurée pour monter et convoyer la cellule d'écoulement (800).
